# FASCICULE DE BREVET EUROPEEN

(11) **EP 0 434 858 B1**
(45) Date de publication et mention de la délivrance du brevet: **23.02.1994**
(21) Numéro de dépôt: 89123930.3
(22) Date de dépôt: 27.12.1989
(51) Int. Cl.: B01J 20/32, G01N 33/548

(54) **Produit de réaction d'un dextranomère greffé et d'un colorant phthalocyanine et son utilisation**
Reaktionsprodukt eines gepfropften Dextranomeres und eines Phthalocyaninfarbstoffs und dessen Verwendung
Reaction product of a grafted dextranomer and a phthalocyanin dye and its use

(43) Date de publication de la demande: 03.07.1991
(73) Titulaire: SOCIETE DES PRODUITS NESTLE S.A., CH-1800 Vevey (CH)
(72) Inventeur: Gross, Gian-Andrea, CH-1633 Marsens (CH)

(56) Documents cités:
- EP-A- 0 064 833
- EP-A- 0 090 610
- US-A- 4 490 525

## Description

L'invention concerne un produit de réaction d'un dextranomère greffé et d'un colorant phthalocyanine. L'invention concerne en outre l'utilisation de ce produit de réaction pour adsorber sélectivement des amines hétérocycliques mutagènes.

Depuis un certain temps, les substances mutagènes présentes dans notre environnement et un certain nombre de nos aliments sont devenues un sujet de préoccupation majeur en raison de leur implication sur la santé de l'homme en tant que cancérigènes potentiels. Il y a donc un besoin important de disposer de techniques permettant d'analyser et d'éliminer de telles substances.

Il est connu que les composés du type phthalocyanines jouissent de la propriété de former des complexes stables avec les substances mutagènes polycycliques. Cette propriété a été utilisée pour préparer des produits d'adsorption sélective de substances mutagènes polycycliques, contenues dans des milieux aqueux essentiellement, en faisant réagir des phthalocyanines à groupe réactif avec des matériaux solides organiques ou inorganiques. Par exemple, le brevet EP 90 610 fait état d'une méthode pour adsorber les substances mutagènes polycycliques utilisant un produit d'adsorption préparé à partir de cellulose et d'un colorant phthalocyanine à groupe réactif. Dans le brevet US 4,490,525, on prépare des produits d'adsorption des substances mutagènes polycycliques à partir de polysaccharides du type agarose ou dextrine, au préalable activés chimiquement, sur lesquels on greffe des phthalocyanines réactives. Dans la demande EP 157 549, des phthalocyanines contenant un groupe réactif sont fixées chimiquement à du gel de silice sur lequel sont greffés des résidus aminopropyle. Cependant, avec toutes ces matières adsorbantes pour les substances mutagènes polycycliques citées en exemple, l'affinité à l'endroit desdites substances mutagènes est conférée presque exclusivement par le résidu phthalocyanine et ne fait pas appel à des propriétés intrinsèques d'affinité pour lesdites substances mutagènes que pourrait avoir le matériau solide de départ utilisé pour la préparation du produit adsorbant. De surcroît, ces matières n'ont pas d'affinité que pour les substances mutagènes polycycliques mais également pour un certain nombre d'autres substances susceptibles d'être aussi présentes dans les milieux contenant lesdites substances mutagènes, de sorte que la sélectivité de ces matières adsorbantes vis-à-vis desdites substances mutagènes est souvent médiocre. D'autre part, les matières adsorbantes citées en exemple n'opèrent que lorsque les substances mutagènes sont contenues dans des milieux essentiellement aqueux. Elles ne peuvent par conséquent pas être utilisées dans tous les cas où la mise en contact avec les substances mutagènes devrait avoir lieu dans un solvant organique.

Il est connu de la littérature que d'autres matières adsorbantes que celles contenant des résidus de phthalocyanine présentent une certaine affinité pour les substances mutagènes polycycliques. Par exemple, G.A. Gross et al., dans Carcinogenesis 1989, 10(7), 1175-1182, ont montré qu'une matière préparée à partir de dextranomère, qui est un polymère de type dextrane réticulé par des ponts 2-hydroxy-1,3-propylène et sur lequel sont greffés des groupes hydroxypropyle, ci-après dénommé dextranomère greffé, montre une sélectivité d'adsorption des substances mutagènes polycycliques supérieure à celle de la plupart des autres matières adsorbantes connues ne contenant pas de résidus phthalocyanine.

Par exemple, l'exploitation de ce dextranomère greffé à des fins analytiques permet de simplifier et d'augmenter la précision des méthodes de dosage des substances mutagènes polycycliques dans des produits alimentaires. Dans certains cas cependant, lorsque par exemple ces substances mutagènes sont contenues dans des milieux d'une grande complexité chimique ou que dans ces milieux leur concentration est particulièrement faible, la sélectivité de ce dextranomère greffé vis-à-vis desdites substances mutagènes peut n'être plus suffisante pour permettre de détecter lesdites substances par des méthodes analytiques.

L'invention concerne un produit de réaction d'un dextranomère greffé comportant des groupements hydroxypropyle et d'un colorant phthalocyanine comportant un atome métallique ou non, portant un groupement réactif capable de réagir avec le groupement hydroxy dudit dextranomère greffé en formant une liaison covalente avec celui-ci. L'atome métallique est choisi parmi les atomes de cuivre, fer, nickel, cobalt, et aluminium. Cet atome est de préférence l'atome de cuivre.

Le produit de réaction selon l'invention est capable d'adsorber sélectivement les substances mutagènes et carcinogènes polycycliques en solution dans un milieu aqueux ou organique. Selon l'invention, on a trouvé que lorsqu'on lie chimiquement une phthalocyanine possédant un groupe réactif à du dextranomère greffé, on obtient un produit de réaction possédant à la fois une capacité d'adsorption des substances mutagènes polycycliques, une sélectivité à l'égard de ces substances et une capacité de restitution de ces substances par désorption tout à fait remarquables.

Dans la pratique, on prépare le produit de réaction selon l'invention en faisant réagir les fonctions hydroxyle du dextranomère greffé avec le groupe réactif de la phthalocyanine selon les procédures de fixation de colorants à des matériaux fibreux familières à l'industrie des colorants. Des exemples de tels groupes réactifs de phthalocyanines pouvant être utilisés pour cette réaction sont les groupes dihalotriazinyle, monohalotriazinyle, trihalopyrimidinyle, sulfatoéthylsulfonyle, chloroéthylsulfonyle, dihaloquinoxalinyle, dihalopyridazonyle, sulfatoéthylsulfonamidyle, mono- ou dihalopyrimidinyle, dihalophthalazinyle, acrylamidyle, vinylsufonyle, halobenzothiazolyle, méthylolamino et β -sulfatoéthylsulfonylphénylaminosulfonyle.

Comme phthalocyanines à groupe réactif, on utilisera avantageusement les colorants phthalocyanines réactifs bien connus de l'industrie des colorants.

Comme dextranomère greffé, bien qu'on puisse aisément préparer cette matière à partir du dextranomère en y greffant sur les fonctions hydroxyle des résidus hydroxypropyle, on préférera dans la pratique utiliser des matières finies disponibles dans le commerce, comme par exemple celles fabriquées par Pharmacia Fine Chemicals AB, Uppsala (Suède), dont la structure unitaire est illustrée ci-dessous: 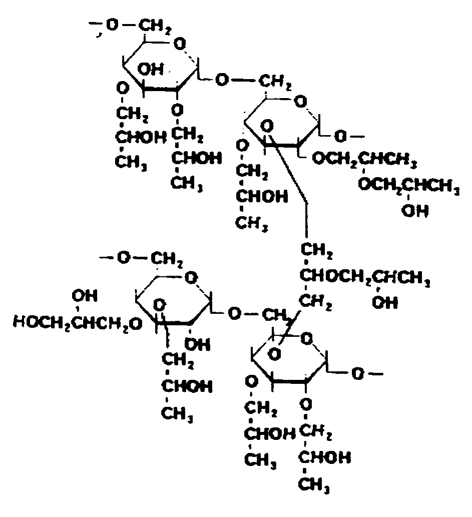
Dans une forme préférée du produit selon l'invention, le dextranomère greffé sera le produit de Pharmacia et le colorant phthalocyanine réactif sera le Bleu Réactif 21 de l'Index des Couleurs (C.I. Reactive Blue 21), par exemple commercialisé par Hoechst AG, Francfort ( Allemagne).

Les substances mutagènes auxquelles la présente invention fait référence sont des composés aromatiques ou hétérocycliques possédant au moins deux noyaux accolés. Comme exemple de telles substances mutagènes on peut citer les composés suivants:

Trp-P-1 (3-amino-1,4-diméthyl-5H-pyrido[4,3-b]indole), Trp-P-2 (3-amino-1-méthyl-5H-pyrido[4,3-b]indole), Glu-P-1 (2-amino-6-méthyl-dipyrido[1,2-a:3',2'-d]imidazole, Glu-P-2 (2-amino-dipyrido[1,2-a:3',2'-d]imidazole, amino-α-carboline (2-amino-9H-pyrido[2,3-b]indole), amino-méthyl-α-carboline (2-amino-3-méthyl-9H-pyrido[2,3-b] indole), IQ (2-amino-3-méthylimidazo[4,5-f] quinoline, 2-acétylaminofluorène, bromure d'éthidium, MeIQ_{X} (2-amino-3,8-diméthylimidazo[4,5-f] quinoxaline), 9-aminoacridine, quinacrine, 8-méthoxypsoralène, chloropromazine, harmane, norharmane (β-carboline), MeIQ (2-amino-3,4-diméthylimidazo [4,5-f] quinoline), 4,8-DiMeIQx (2-amino-3,4,8-triméthylimidazo [4,5-f] quinoxaline, 7,8-DiMeIQx (2-amino-3,7,8-triméthylimidazo [4,5-f] quinoxaline, 4,7,8 - TriMeIQx (2-amino-3,4,7,8-tétraméthylimidazo [4,5-f] quinoxaline et benzo [a] pyrène.

La présente invention concerne en outre l'utilisation des produits de réaction consistant à adsorber sélectivement sur ledit produit de réaction des substances organiques polycycliques, en particulier des amines hétérocycliques, en solution dans des milieux aqueux ou organiques. L'invention permet d'éliminer ou d'analyser les substances mutagènes polycycliques usuellement présentes en quantités infimes (ppb) dans des produits destinés à la consommation ou dans des milieux biologiques.

Si on souhaite éliminer les substances mutagénes, on effectue l'adsorption sélective sur le produit de réaction et soit on jette la colonne d'adsorption, soit on la régénère en éluant les substances mutagènes. Si on souhaite analyser ces substances mutagènes, on fait une adsorption sélective et on désorbe lesdits produits mutagènes pour les récupérer sous une forme concentrée.

De plus, la capacité d'adsorption et la sélectivité se manifestent non seulement lorsque les substances mutagènes sont présentes dans un milieu aqueux, mais également lorsqu'elles sont présentes dans un solvant organique, entendant par solvant organique non seulement les solvants polaires ou protiques, comme par exemple le méthanol, l'éthanol ou l'acétone, mais également des solvants peu polaires ou apolaires, comme l'acétate d'éthyle, l'éther diéthylique, les solvants halogénés ou les hydrocarbures volatils. Comme solvant organique on utilise de préférence le dichlorométhane.

La mise en contact du produit d'adsorption selon l'invention avec les substances mutagènes polycycliques se fait en ajoutant le dextranomère greffé à une solution, aqueuse ou organique, contenant lesdites substances mutagènes et en agitant ce mélange à température ambiante. Dans une forme préférée de mise en contact, le produit d'adsorption est rempli dans une colonne à travers laquelle on fait ensuite passer une solution aqueuse ou organique contenant les substances mutagènes.

Si on le désire, la désorption des substances mutagènes du produit de réaction selon l'invention pourra être réalisée en mettant en contact le produit de réaction sur lequel sont adsorbées les substances mutagènes avec un solvant neutre ou légèrement acide ou alcalin, comme par exemple du méthanol ou un mélange de méthanol avec de l'acide acétique ou de l'acide trifluoroacétique, ou mélange de méthanol avec de l'ammoniac. Cette mise en contact pourra se faire soit par immersion du produit d'adsorption dans le solvant de désorption, soit de préférence en passant le solvant de désorption à travers une colonne contenant le produit d'adsorption.

La capacité du produit de réaction selon l'invention à adsorber sélectivement et à restituer les substances mutagènes polycycliques peut être exploitée dans la mise en oeuvre de méthodes visant à éliminer ou à analyser ces substances mutagènes, par exemple celles que l'on peut trouver dans certains aliments, dans la fumée de tabac, dans les eaux destinées à la consommation ou dans des milieux biologiques.

Dans les exemples qui suivent, la présente invention est illustrée de manière plus détaillée, étant entendu que ces exemples ne sont pas limitatifs de la portée de l'invention.

### Exemple 1: Préparation du produit de réaction selon l'invention

On chauffe 100 g de dextramère greffé à 80°C pendant 2 heures dans une solution de 5 g de bleu de turquoise, 12 g de Carbonate de sodium et 30 g de sulfate de sodium dissouts dans 600 ml d'eau. On laisse refroidir le mélange et le produit filtré est lavé avec 1 l d'eau et ensuite avec 1 l d'un mélange 1:1 de méthanol et d'ammoniaque concentré. Le produit final est ensuite lavé en continu avec 7 l de méthanol et il est finalement essoré à fond et séché sous vide. On obtient ainsi le produit selon l'invention que l'on appelle par la suite dextramère greffé bleu.

Le produit est caractérisé par son contenu en ions de cuivre par spectrophotométrie d'adsorption atomique. La teneur en cuivre est de 520µg/g de dextramère greffé bleu.

### Exemple 2: Capacité d'adsorption des substances mutagènes polycycliques par le Sephasorb bleu.

On charge une colonne d'extraction de 2 ml avec le produit de réaction selon l'exemple 1, mis en suspension dans une solution méthanolique à un volume de 0,5 ml de Sephasorb bleu. On relie l'extrémité inférieure de la colonne par un tube de polytetrafluoroéthylène à un enregistreur UV réglé pour une lecture à 254 nm. La colonne est rincée avec un peu de méthanol, puis on passe du dichlorométhane (DCM) jusqu'à ce qu'on atteigne une ligne de base stable sur l'enregistreur. Une solution 10⁻⁵ Molaire d'une substance mutagène dans le DCM est passée sur la colonne à un débit de 0,3 ml/min. jusqu'à ce qu on atteigne la saturation du dextramère greffé bleu par la substance mutagène que l'on mesure sur l'enregistreur au point d'inflexion de la courbe d'adsorption (voir Liska et al, J. High Res. Chrom.,1989, 12, 577-590).

On a une capacité d'adsorption de IQ par le produit de réaction selon l'invention de 200µg par ml de dextramère greffé bleu.

Si on répète l'opération avec une colonne de dextramère greffé non traité, on arrive à une valeur inférieure à 10µg par ml de dextramère greffé.

### Exemple 3: Application du produit selon l'invention pour une méthode d'analyse des substances polycycliques présentes dans des extraits de viande.

Une fraction basique d'extrait de viande préparée à partir d'un g d'extrait de viande (selon la méthode décrite par G.A. Gross et al. dans l'article mentionné page 2) contenue dans 10 ml de DCM est passée sur une colonne de 4 ml contenant 1 ml de dextramère greffé bleu à un débit de 0,4 ml/min. On rince la colonne avec 5 ml de DCM. On désorbe avec 10 ml d'une solution de DCM à 15% de méthanol et 0,1% d'ammoniaque concentré. L'eluat est évaporé à sec.

Pour la suite on peut opérer selon deux méthodes. Soit on dissout le résidu solide dans 100µl de méthanol, on filtre sur un microfiltre et on injecte un aliquote de cette solution sur un chromatographe HPLC avec détection UV.

Soit, si on veut avoir une meilleure sensibilité de mesure (en-dessous de 0,1 ppm), on dissout le résidu dans 100µl de méthanol et on complète avec 2 ml d'eau. On lave cette solution sur une colonne de dextramère greffé et on évapore à sec. On reprend ensuite le résidu dans 100µl de méthanol, on filtre sur microfiltre et on injecte la solution sur un chromatographe HPLC.

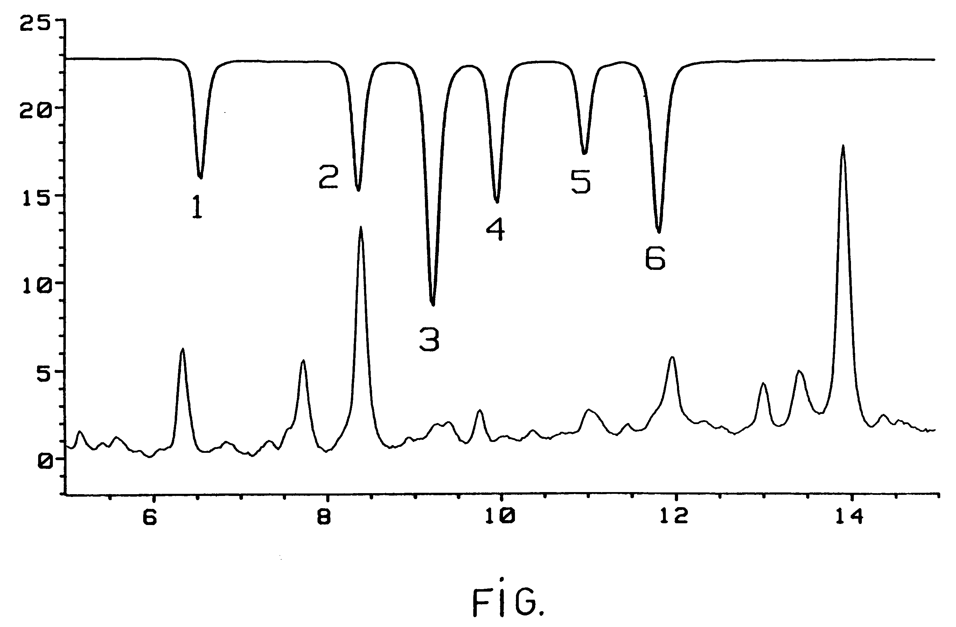

La figure ci-jointe montre un chromatogramme selon cette seconde méthode. La courbe supérieure donne le chromatogramme d'une substance de référence et celle du bas celui de l'extrait de viande analysé. L'abscisse donne le temps en min. et l'ordonnée la teneur en composé mutagène.

Le composé 1 est IQx, le composé 2 MeIQx, 3=IQ, 4=7,8-DiMeIQx, 5=4,8-DiMeIQx et 6=MeIQ.

## Revendications

1. Produit de réaction d'un polymère de dextrane réticulé par des ponts 2-hydroxy-1,3 propylène et sur lequel sont greffés des groupements hydroxypropyle et d'un colorant phthalocyanine avec un atome métallique ou non portant un groupement réactif susceptible de réagir avec le groupement hydroxy dudit polymère de dextrane.

2. Produit de réaction selon la revendication 1, caractérisé en ce que l'atome métallique de la phthalocyanine est choisi parmi les atomes de cuivre, fer, nickel, cobalt et aluminium.

3. Produit de réaction selon la revendication 1, caractérisé en ce que le colorant phthalocyanine est le bleu de turquoise, l'atome métallique étant le cuivre.

4. Utilisation du produit de réaction selon les revendications 1 à 3 comme adsorbant de composés mutagènes, caractérisé en ce qu'on met en contact le produit adsorbant avec une phase aqueuse ou organique contenant les produits mutagènes, ceux-ci étant adsorbés sélectivement sur ledit produit de réaction.

5. Utilisation selon la revendication 4, caractérisé en ce que la phase organique contenant les produits mutagènes est à base de dichlorométhane.

6. Utilisation selon l'une des revendications 4 et 5, caractérisé en ce qu'on désorbe les produits mutagènes du produit de réaction.

## Claims

1. A reaction product of a dextrane monomer, which is crosslinked by 2-hydroxy-1,3-propylene bridges and onto which hydroxypropyl groups are grafted, and a phthalocyanine dye optionally containing a metal atom and bearing a reactive group capable of reacting with the hydroxy group of the dextrane polymer.

2. A reaction product as claimed in claim 1, characterized in that the metal atom of the phthalocyanine is selected from copper, iron, nickel, cobalt and aluminium atoms.

3. A reaction product as claimed in claim 1, characterized in that the phthalocyanine dye is turquoise blue, the metal atom being copper.

4. The use of the reaction product claimed in claims 1 to 3 as an adsorbent for mutagenic compounds, characterized in that the adsorbent is contacted with an aqueous or organic phase containing the mutagenic products, the mutagenic products being selectively adsorbed onto the reaction product.

5. The use claimed in claim 4, characterized in that the organic phase containing the mutagenic products is based on dichloromethane.

6. The use claimed in claim 4 or 5, characterized in that the mutagenic products are desorbed from the reaction product.

## Patentansprüche

1. Reaktionsprodukt eines durch 2-Hydroxy-1,3-propylen-Brücken vernetzten Dextranpolymers, auf das Hydroxypropylgruppen aufgepfropft sind, und eines Phthalocyaninfarbstoffs mit oder ohne Metallatom, der eine reaktive Gruppe trägt, die mit der Hydroxygruppe des Dextranmolymers reagieren kann.

2. Reaktionsprodukt nach Anspruch 1, dadurch gekennzeichnet, daß das Metallatom des Phthalocyanins aus Kupfer-, Eisen-, Nikkel-, Kobalt- und Aluminiumatomen ausgewählt ist.

3. Reaktionsprodukt nach Anspruch 1, dadurch gekennzeichnet, daß der Phthalocyaninfarbstoff Türkisblau ist, wobei das Metallatom Kupfer ist.

4. Verwendung des Reaktionsprodukts nach den Ansprüchen 1 bis 3 als Mittel zur Adsorption von mutagenen Verbindungen, dadurch gekennzeichnet, daß das adsorbierende Produkt mit einer wässrigen oder organischen, die mutagenen Produkte enthaltenden Phase in Kontakt gebracht wird, wobei diese Produkte auf dem Reaktionsprodukt selektiv adsorbiert werden.

5. Verwendung nach Anspruch 4, dadurch gekennzeichnet, daß die die mutagenen Produkte enthaltende organische Phase eine auf der Basis von Dichlormethan ist.

6. Verwendung nach den Ansprüchen 4 und 5, dadurch gekennzeichnet, daß die mutagenen Produkte von dem Reaktionsprodukt desorbiert werden.
